# EUROPÄISCHE PATENTANMELDUNG

(11) **EP 2 269 677 A2**
(43) Veröffentlichungstag der Anmeldung: **05.01.2011**
(21) Anmeldenummer: 10181552.0
(22) Anmeldetag: 24.10.2000
(51) Int. Cl.: A61M 16/06

(54) **Atemmaske und Verfahren zur Herstellung derselben**

(30) Priorität: 12.11.1999 DE 19954517
(62) Teilanmeldung aus: 00122358.5
(71) Anmelder: MAP Medizin-Technologie GmbH, 82152 Martinsried (DE)
(72) Erfinder: Lang, Bernd Christoph, 82166 Lochham (DE); Genger, Harald, 82319 Starnberg (DE)
(74) Vertreter: Vossius & Partner

(57) **Zusammenfassung**

Die Erfindung betrifft eine Atemmaske zur Zufuhr eines Atemgases zu einem Patienten sowie ein Verfahren zur Herstellung derselben. Die erfindungsgemäße Atemmaske umfaßt eine nachgiebige Dichtungseinrichtung zur Abdichtung eines in einem Maskenbasiskörper gebildeten Maskeninnenraumes. Die Dichtungseinrichtung ist mit einem gelartig ausgebildeten Abschnitt versehen, der mit einer Hautschicht überzogen ist, die ebenfalls aus einem Elastomermaterial gebildet ist. Insbesondere auch alternativ hierzu kann die Dichtungseinrichtung ein Luftpolsterelement aufweisen. Nach dem erfindungsgemäßen Verfahren wird der Maskenbasiskörper im Rahmen eines Spritzvorganges an die Dichtungseinrichtung angespritzt.

## Beschreibung

Die Erfindung betrifft eine Atemmaske zur Zufuhr eines Atemgases zu einem Patienten sowie ein Verfahren zur Herstellung derselben.

Atemmasken der eingangs genannten Art finden insbesondere Anwendung im Bereich der Schlafmedizin. Über derartige Atemmasken kann dem Patienten ein Atemgas Umgebungsluft unter einem vorbestimmten Überdruck zugeführt werden. Hierdurch wird auf physiologisch vergleichsweise gut verträgliche Weise eine pneumatische Schienung der oberen Atemwege erreicht wodurch etwaigen Obstruktionen in diesem Bereich auf vorteilhafte Weise vorgebeugt werden kann.

Die im Bereich der Schlafmedizin angewandten Masken werden bei der Behandlung eines Patienten im Rahmen einer CPAP-Schlaftherapie täglich ca. 6 bis 8 Stunden getragen. Bei der Anwendung der bislang bekannten Masken besteht bei vielen Patienten das Problem, daß die gewünschte Dichtwirkung der Maske nur unter vergleichsweise großen Masken-Haltekräften erreicht werden kann und hierbei der Tragekomfort u.U. erheblich beeinträchtigt wird.

Der Erfindung liegt die Aufgabe zugrunde, eine Atemmaske zur Zufuhr eines Atemgases unter Überdruck zu schaffen die sich durch einen hohen Tragekomfort auszeichnet und durch welche ein hoher Abdichtungsgrad auf zuverlässige Weise gewährleistet ist.

Diese Aufgabe wird erfindungsgemäß jeweils für sich durch eine Atemmaske mit wenigstens den in den unabhängigen Patentansprüchen und angegebenen Merkmalen gelöst.

Dadurch wird es auf vorteilhafte Weise möglich bei einer geringen Flächenpressung im Bereich der Gesichtskontaktzone durchgängig eine innige Anlage des inneren Dichtungsbereiches zu erreichen ohne daß die Gefahr besteht daß kleinere Positionsänderungen der Atemmaske gegenüber dem Gesicht des Patienten zu erheblichen Änderungen der Anpreßkräfte führen. In vorteilhafter Weise ist auch eine gegenüber herkömmlichen Atemmasken bessere Kompatibilität zu den individuellen Gesichtsformen der jeweiligen Patienten gegeben.

Eine sowohl im Hinblick auf einen hohen Tragekomfort sowie auch unter fertigungstechnischen Gesichtspunkten vorteilhafte Ausführungsform der Erfindung ist dadurch gegeben, daß der Maskenbasiskörper aus einem elastomeren Material gebildet ist. Im Hinblick auf eine hohe physiologische Verträglichkeit sowie gute Sterilisierbarkeit sind der Maskenbasiskörper und das Stirnauflageelement vorzugsweise aus einem Silikonmaterial gebildet. Auch die Dichtungseinrichtung ist vorzugsweise aus einem Silikonmaterial gebildet.

Eine besonders belastbare und im wesentlichen spaltfreie Koppelung von Maskenkörper und Dichtungseinrichtung ist gemäß einer besonders bevorzugten Ausführungsform der Erfindung dadurch gegeben, daß Maskenbasiskörper an die Dichtungseinrichtung angespritzt ist. Alternativ dazu ist es auch möglich, die Dichtungseinrichtung an den Maskenbasiskörper anzuspritzen sofern das zur Bildung des Maskenkörpers verwendete Material eine hinreichende Temperaturbeständigkeit aufweist.

Eine im Hinblick auf einen besonders hohen Tragekomfort sowie im Hinblick auf eine hohe Dichtwirkung vorteilhafte Ausführungsform der Erfindung ist dadurch gegeben, daß ein Versteifungselement vorgesehen ist, das dem Maskenbasiskörper und/oder der Dichtungseinrichtung eine vorbestimmte Gestalt verleiht. Dieses Versteifungselement ist vorzugsweise aus einem unter Wärmezufuhr verformbaren Material gebildet und behält nach Erkalten auf Raumtemperatur die ihm verliehene Gestalt unter allenfalls elastischer Verformung weitgehend bei.

Eine besonders individuelle Anpassung der Atemmaske an die Kopfform des Patienten wird gemäß einer besonders bevorzugten Ausführungsform der Erfindung dadurch erreicht, daß sich das Versteifungselement bis in das Stirnauflageelement hinein erstreckt.

In dem Maskenbasiskörper und/oder dem Stirnauflageelement ist vorzugsweise ein Aufnahmeabschnitt ausgebildet, zur Aufnahme des Versteifungselementes. Dieser Aufnahmeabschnitt kann durch eine komplementär zum Querschnitt des Versteifungselementes ausgebildete Ausnehmung gebildet sein. Vorzugsweise ist das Versteifungselement eng sitzend in dieser Ausnehmung aufgenommen, bedarfsweise eingeklebt.

An dem Stirnauflageelement ist vorzugsweise eine Koppelungseinrichtung vorgesehen, zum Anschluß eines Maskenhaltebandes.

Eine im Hinblick auf eine besonders gleichmäßige Verteilung der Maskenhaltekräfte vorteilhafte Ausführungsform der Erfindung ist dadurch gegeben, daß die Dichtungseinrichtung ein Polsterorgan aufweist das von einem elastomeren Wandungsmaterial begrenzt ist. Das Polsterorgan erstreckt sich vorzugsweise in der Art eines Schlauchpolsters entlang des Maskenauflagebereiches. An dem Polsterorgan ist vorzugsweise eine einstückig mit dem Polsterorgan ausgebildete Dichtlippe vorgesehen die sich vom Maskenaußenbereich einwärts erstreckt.

Insbesondere die Kombination aus Schlauchpolster und integralem Stirnauflageelement, ggf. mit Versteifungseinlage, führt zu einer Atemmaske, die sich bei hoher Dichtigkeit durch einen ausnehmend hohen Tragekomfort auszeichnet.

Im Hinblick auf eine besonders einfache Reinigungsmöglichkeit ist vorzugsweise ein Innenübergangsbereich zwischen der Dichtlippe und dem Polsterorgan gerundet ausgebildet.

Die Dichtungseinrichtung zur Abdichtung des Maskenbasiskörpers kann alternativ zu dem beschriebenen Polsterkörper auch ein aus einem gelartig ausgehärteten Elastomermaterial gebildetes Organ aufweisen, wobei der Elastomermaterial-Abschnitt vorzugsweise mit einer Hautschicht überzogen ist die aus einem elastomeren Material gebildet ist. Hierdurch wird auf überraschend wirkungsvolle Weise einer ungünstigen Bildung von Falten vorgebeugt. Die Hautschicht ist vorzugsweise aus einem höher vernetzten Silikonkautschukmaterial gebildet.

Die genannte Hautschicht kann auf fertigungstechnisch vorteilhafte Weise durch Auftrag einer dünnen Silikonmaterialschicht auf einen entsprechenden Formraumabschnitt eines Formwerkzeuges gebildet werden, wobei später das gelartig aushärtende Silikonmaterial in den entsprechend vorbereiteten, verbleibenden Formraumabschnitt eingespritzt ist und hierbei eine innige Verbindung mit dem Hautabschnitt erreicht wird.

Ggf. auch unabhängig von den vorangehend beschriebenen Maßnahmen ist eine gemäß einem weiteren Erfindungsgedanken insbesondere unter fertigungstechnischen Gesichtspunkten vorteilhafte Ausführungsform der Atemmaske mit einem Maskenbasiskörper der einen Maskeninnenraum begrenzt, einem Atemgaszuleitungsabschnitt zur Zuleitung von Atemgas zu dem Maskeninnenraum, und einer nachgiebigen Dichtungseinrichtung zur Abdichtung des Maskenbasiskörpers dadurch gegeben, daß der Maskenbasiskörper aus einem thermoplastischen Kunststoffmaterial gebildet und an die Dichtungseinrichtung angespritzt ist.

Weiterhin ist es in vorteilhafter Weise möglich, die Dichtungseinrichtung derart auszubilden, daß diese ein sich entlang eines Maskenauflagebereiches erstreckendes Luftpolsterelement umfaßt und daß eine Naht- oder Klebestelle des Luftpolsterelementes zumindest abschnittsweise von einem Aufnahmeabschnitt des Maskenbasiskörpers übergriffen ist.

Ein im Hinblick auf einen hohen Tragekomfort besonders vorteilhaftes Polsterelement wird insbesondere auch unabhängig von den vorangehend beschriebenen Maßnahmen dadurch erreicht, daß die Dichtungseinrichtung ein Dichtiippenelement aufweist das aus einem Silikonmaterial mit hohem Vernetzungsgrad gefertigt ist, und daß an dieses Dichtlippenelement ein Polsterkörper angekoppelt ist, der aus einem Silikonmaterial niedrigen

Vernetzungsgrades gefertigt ist. Vorzugsweise ist der Polsterkörper zumindest im Bereich der Gesichtsauflagefläche mit einer elastomeren Haut überzogen.

Insbesondere bei der Ausgestaltung des Polsterelementes als Luftpolster wird gemäß einer besonders bevorzugten Ausführungsform der Erfindung die Dichtungseinrichtung durch ein aus einem elastomeren Material gebildetes Schlauch-Polster gebildet wobei Wandungsabschnitte des Schlauchpolsters derart definiert dick- und dünnwandig ausgebildet sind, daß in vorbestimmten Zonen Gelenkzonen entstehen.

Vorzugsweise ist bei dieser Ausführungsform ein einwärts gerichtet umlaufendes Dichtlippenelement einstückig mit dem Polsterkörper ausgebildet.

Unter fertigungstechnischen Gesichtspunkten wird die eingangs angegebenen Aufgabe auch durch ein Verfahren zur Herstellung einer Atemmaske gelöst, bei welchem im Rahmen eines ersten Silikoneinspritzvorganges Silikonmaterial in einen Formraumabschnitt eingespritzt wird der komplementär zu einer Masken-Dichtungseinrichtung ausgebildet ist, wobei im Rahmen eines nachfolgenden Verfahrensschrittes ein Maskenkörperseitiger Verbindungsbereich zunächst entformt und anschließend in einen komplementär zu einem Maskenkörper ausgebildeten Formraumabschnitt eingebracht wird, und erst anschließend ein Kunststoffmaterial in den, zum Maskenkörper komplementären Formraum eingespritzt wird.

Die folgenden Punkte sind bevorzugte Ausführungsformen der vorliegenden Erfindung.
**1.** Atemmaske mit:
   - einem Maskenbasiskörper der einen Maskeninnenraum begrenzt,
   - einem Atemgaszuleitungsabschnitt zur Zuleitung von Atemgas zu dem Maskeninnenraum, und
   - einer nachgiebigen Dichtungseinrichtung zur Abdichtung des Maskenbasiskörpers **dadurch gekennzeichnet, daß** der Maskenbasiskörper mit einem integral ausgebildeten Stirnauflageelement versehen ist.
**2.** Atemmaske nach Punkt 1, **dadurch gekennzeichnet, daß** der Maskenbasiskörper aus einem elastomeren Material gebildet ist.
**3.** Atemmaske nach Punkt 1 oder 2, **dadurch gekennzeichnet, daß** der Maskenbasiskörper und das Stirnauflageelement aus einem Silikonmaterial gebildet sind.
**4.** Atemmaske nach wenigstens einem der Punkte 1 bis 3, **dadurch gekennzeichnet, daß** die Dichtungseinrichtung aus einem Silikonmaterial gebildet ist.
**5.** Atemmaske nach wenigstens einem der Punkte 1 bis 4, **dadurch gekennzeichnet, daß** der Maskenbasiskörper an die Dichtungseinrichtung angespritzt ist.
**6.** Atemmaske nach wenigstens einem der Punkte 1 bis 5, **dadurch gekennzeichnet, daß** die Dichtungseinrichtung an den Maskenbasiskörper angespritzt ist.
**7.** Atemmaske nach wenigstens einem der Punkte 1 bis 6, **dadurch gekennzeichnet, daß** ein Versteifungselement vorgesehen ist, das dem Maskenbasiskörper und/oder der Dichtungseinrichtung eine vorbestimmte Gestalt verleiht.
**8.** Atemmaske nach wenigstens einem der Punkte 1 bis 7, **dadurch gekennzeichnet, daß** das Versteifungselement aus einem unter Wärmezufuhr verformbaren Material gebildet ist und bei Erkalten auf Raumtemperatur die im verliehene Gestalt weitgehend beibehält.
**9.** Atemmaske nach wenigstens einem der Punkte 1 bis 8, **dadurch gekennzeichnet, daß** sich das Versteifungselement bis in das Stirnauflageelement hinein erstreckt.
**10.** Atemmaske nach wenigstens einem der Punkte 1 bis 9, **dadurch gekennzeichnet, daß** in dem Maskenbasiskörper und/oder dem Stirnauflageelement ein Aufnahmeabschnitt ausgebildet ist, zur Aufnahme des Versteifungselementes.
**11.** Atemmaske nach wenigstens einem der Punkte 1 bis 10, **dadurch gekennzeichnet, daß** das Stirnauflageelement mit einer Koppelungseinrichtung versehen ist, zum Anschluß eines Maskenhaltebandes.
**12.** Atemmaske nach wenigstens einem der Punkte 1 bis 11, **dadurch gekennzeichnet, daß** die Dichtungseinrichtung ein Polsterorgan aufweist das aus einem elastomeren Wandungsmaterial begrenzt ist.
**13.** Atemmaske nach wenigstens einem der Punkte 1 bis 12, **dadurch gekennzeichnet, daß** das Polsterorgan sich in der Art eines Schlauchpolsters entlang des Maskenaufiagebereiches erstreckt.
**14.** Atemmaske nach wenigstens einem der Punkte 1 bis 13, **dadurch gekennzeichnet, daß** eine einstückig mit dem Polsterorgan ausgebildete Dichtlippe vorgesehen ist die sich vom Maskenaußenbereich einwärts erstreckt.
**15.** Atemmaske nach wenigstens einem der Punkte 1 bis 14, **dadurch gekennzeichnet, daß** ein Innenübergangsbereich zwischen der Dichtlippe und dem Polsterorgan gerundet ausgebildet ist.
**16.** Atemmaske mit:
   - einem Maskenbasiskörper der einen Maskeninnenraum begrenzt,
   - einem Atemgaszuleitungsabschnitt zur Zuleitung von Atemgas zu dem Maskeninnenraum, und
   - einer nachgiebigen Dichtungseinrichtung zur Abdichtung des Maskenbasiskörpers wobei die Dichtungseinrichtung ein aus einem gelartig ausgehärteten Elastomermaterial gebildeten Abschnitt aufweist, **dadurch gekennzeichnet, daß** der Elastomermaterial-Abschnitt mit einer Hautschicht überzogen ist die aus einem elastomeren Material gebildet ist.
**17.** Atemmaske nach Punkt 16, **dadurch gekennzeichnet, daß** die Hautschicht aus einem höher vernetzten Silikonkautschukmaterial, insbesondere durch einen Tauchvorgang gebildet ist.
**18.** Atemmaske nach Punkt 16 oder 17, **dadurch gekennzeichnet, daß** die Hautschicht durch Auftrag einer dünnen Silikonmaterialschicht auf einen entsprechenden Formraumabschnitt eines Formwerkzeuges gebildet ist, und daß das gelartig aushärtende Silikonmaterial in den entsprechend vorbereiteten Formraumabschnitt eingespritzt ist.
**19.** Atemmaske mit:
   - einem Maskenbasiskörper der einen Maskeninnenraum begrenzt,
   - einem Atemgaszuleitungsabschnitt zur Zuleitung von Atemgas zu dem Maskeninnenraum, und
   - einer nachgiebigen Dichtungseinrichtung zur Abdichtung des Maskenbasiskörpers **dadurch gekennzeichnet, daß** der Maskenbasiskörper aus einem thermoplastischen Kunststoffmaterial gebildet und an die Dichtungseinrichtung angespritzt ist.
**20.** Atemmaske mit:
   - einem Maskenbasiskörper der einen Maskeninnenraum begrenzt,
   - einem Atemgaszuleitungsabschnitt zur Zuleitung von Atemgas zu dem Maskeninnenraum, und
   - einer nachgiebigen Dichtungseinrichtung zur Abdichtung des Maskenbasiskörpers **dadurch gekennzeichnet, daß** die Dichtungseinrichtung ein sich entlang eines Maskenauflagebereiches erstreckendes Luftpolsterelement umfaßt und daß eine Nahtoder Klebestelle des Luftpolsterelementes zumindest abschnittsweise von einem Aufnahmeabschnitt des Maskenbasiskörpers übergriffen ist.
**21.** Atemmaske mit:
   - einem Maskenbasiskörper der einen Maskeninnenraum begrenzt,
   - einem Atemgaszuleitungsabschnitt zur Zuleitung von Atemgas zu dem Maskeninnenraum, und
   - einer nachgiebigen Dichtungseinrichtung zur Abdichtung des Maskenbasiskörpers **dadurch gekennzeichnet, daß** die Dichtungseinrichtung ein Dichtlippenelement aufweist das aus einem Silikonmaterial mit hohem Vernetzungsgrad gefertigt ist, und daß an dieses Dichtlippenelement ein Polsterkörper angekoppelt ist, der aus einem Silikonmaterial niedrigen Vernetzungsgrades gefertigt ist.
**22.** Atemmaske nach Punkt 21 **dadurch gekennzeichnet, daß** der Polsterkörper zumindest im Bereich der Gesichtsauflagefläche mit einer elastomeren Haut überzogen ist.
**23.** Atemmaske mit:
   - einem Maskenbasiskörper der einen Maskeninnenraum begrenzt,
   - einem Atemgaszuleitungsabschnitt zur Zuleitung von Atemgas zu dem Maskeninnenraum, und
   - einer nachgiebigen Dichtungseinrichtung zur Abdichtung des Maskenbasiskörpers **dadurch gekennzeichnet, daß** die Dichtungseinrichtung ein aus einem elastomeren Material gebildetes Schlauch-Polsterelement aufweist und daß Wandungsabschnitte des Schlauchpolsters derart definiert dick- und dünnwandig ausgebildet sind, daß definierte Gelenkzonen entstehen.
**24.** Atemmaske nach , Punkt 23, **dadurch gekennzeichnet, daß** ein einwärts gerichtet umlaufendes Dichtlippenelement einstückig mit dem Polsterkörper ausgebildet ist.
**25.** Verfahren zur Herstellung einer Atemmaske bei welchem im Rahmen eines ersten Silikoneinspritzvorganges Silikonmaterial in einen Formraumabschnitt eingespritzt wird der Komplementär zu einer Masken-Dichtungseinrichtung ausgebildet ist, daß im Rahmen eines nachfolgenden Verfahrensschrittes ein maskenkörperseitiger Verbindungsbereich zunächst entformt und anschließend in einen komplementär zu einem Maskenkörper ausgebildeten Formraumabschnitt eingebracht wird, und daß anschließend ein Kunststoffmaterial in den, zum Maskenkörper komplementären Formraum eingespritzt wird.

Weitere Einzelheiten und Merkmale der Erfindung ergeben sich aus der nachfolgenden Beschreibung mehrerer bevorzugter Ausführungsbeispiele in Verbindung mit der Zeichnung. Es zeigen:
- **Fig. 1a:**: eine vereinfachte perspektivische Ansicht einer Atemmaske mit integriertem Stirnauflageelement;
- **Fig. 1b:**: eine vereinfachte Schnittansicht durch eine bevorzugte Ausführungsform ei- nes Luftpolsterbereiches, wie er beispielsweise bei der Atemmaske gem. Fig. 1 a Anwendung finden kann, mit lösbar angebrachtem Versteifungsrahmen;
- **Fig. 1c:**: eine vereinfachte Detailansicht zur Erläuterung eines Befestigungselementes zur Koppelung einer Kopfbandanordnung mit der Atemmaske;
- **Fig. 1d:**: eine vereinfachte perspektivische Ansicht zur Erläuterung des Aufbaus eines Stirnauflageelementes, das integral mit dem Maskenbasiskörper aus einem elastomeren Material gebildet ist;
- **Fig. 1e:**: eine Skizze zur Erläuterung der Gestalt der Maskenpolsterung für den Be- reich des Nasenrückens eines Patienten;
- **Fig. 2:**: eine perspektivische Ansicht eines Versteifungselementes für eine Atemmas- ke, das sich bis in den Stirnauflagebereich hinein erstreckt;
- **Fig. 3a:**: eine weitere Ausführungsform eines Versteifungselementes für einen Mas- kenbasiskörper, das hier aus einem Drahtmaterial mit im wesentlichen kreis- zylindrischem Querschnitt gefertigt ist;
- **Fig. 3b:**: eine perspektivische Detailansicht, durch den Dichtlippenbereich eines Mas- kenelementes im Bereich des Nasenrückens mit daran angebrachtem Ver- steifungselement im wesentlichen gem. Fig. 3a;
- **Fig. 4:**: eine vereinfachte Schnittansicht durch den Dichtlippenbereich einer Atem- maske mit einem Gel-Polsterkörper sowie einem unmittelbar benachbart an- geordneten Versteifungselement;
- **Fig. 5:**: eine vereinfachte Schnittansicht durch einen Dichtlippenbereich mit ange- spritztem Maskenbasiskörper;
- **Fig. 6:**: eine vereinfachte Schnittansicht zur Erläuterung eines Dichtlippenbereiches mit integriertem Schlauchpolster sowie maskenbasiskörperseitig übergriffener Nahtstelle;
- **Fig. 7:**: eine vereinfachte Schnittansicht durch eine Dichtlippenstruktur mit einem an eine Hauptdichtlippe angespritzten Gelkörper dessen dem Patienten zugewandte Außen- seite mit einer elastomeren Hautschicht überzogen ist;
- **Fig. 8:**: eine vereinfachte Schnittansicht durch eine Dichtlippenstruktur mit darin defi- niert ausgebildeten dünnwandigen Wandungszonen zur Bildung definierter Gelenkzonen;

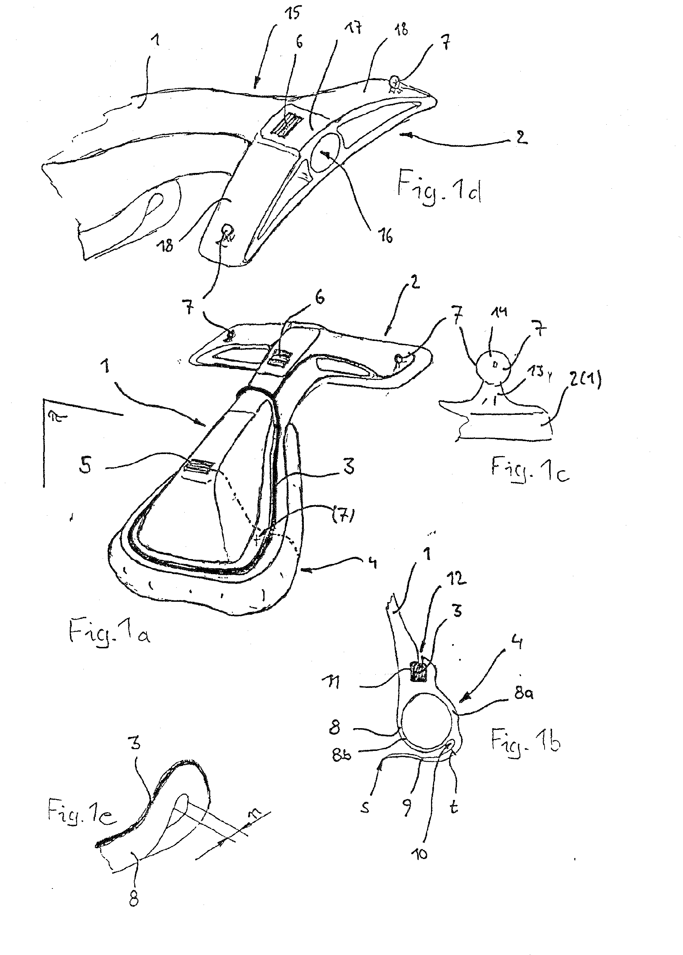

Fig. 1 zeigt eine erste bevorzugte Ausführungsform einer Atemmaske, die einen Maskenbasiskörper 1 mit einem integral damit ausgebildeten Stirnauflageelement 2 aufweist. Bei der dargestellten Ausführungsform sind der Maskenbasiskörper 1 und das Stirnauflageelement 2 aus einem elastomeren Material, insbesondere Silikonkautschuk gebildet. Der Maskenbasiskörper 1 ist durch ein umlaufendes Versteifungselement 3 verstärkt. Bei der gezeigten Ausführungsform besteht das Versteifungselement 3 aus einem Kunststoffmaterial, das hinsichtlich seiner räumlichen Gestalt im erwärmten Zustand individuell auf den Patienten abgestimmt wurde. Das Versteifungselement 3 ist in einer hier nur andeutungsweise dargestellten umlaufenden Nut an dem Maskenbasiskörper 1 fixiert.

Der Maskenbasiskörper 1 weist weiterhin ein integral mit diesem ausgebildetes Dichtlippenelement 4 auf, das hier ein umlaufendes Schlauchpolster (vgl. Fig. 1b) umfaßt. Das Dichtlippenelement 4 und der Maskenbasiskörper 1 sind ebenfalls integral ausgebildet, so daß zwischen dem Dichtlippenelement 4 und dem Maskenbasiskörper keine Spaltbereiche gebildet sind.

In dem Maskenbasiskörper 1 sind weiterhin Auslaßöffnungen 5, 6 gebildet, über weiche permanent unter Überdruck stehendes Gas aus dem Innenbereich der Maske nach außen hin entweichen kann. Die Auslaßöffnungen 5, 6 sind hier durch kleine, in den Maskenbasiskörper 1 eingeformte Schlitze gebildet. Bei der hier dargestellten Ausführungsform sind die Schlitze durch dünne, ebenfalls integral mit dem Maskenbasiskörper 1 gebildete Stege voneinander getrennt. Alternativ hierzu ist es auch möglich, eine Blendenstruktur, die einen definierten Durchgangsquerschnitt aufweist, in eine entsprechende in dem Maskenbasiskörper 1 gebildete Aufnahmeöffnung einzusetzen, insbesondere lösbar einzuklipsen. Die Anordnung der Auslaßöffnungen 5, 6 ist bei der gezeigten Atemmaske derart getroffen, daß im Inneren der Atemmaske keine deutlich wahrnehmbaren Luftströmungen seitens des Patienten auftreten. Der Durchgangsquerschnitt, der dem Stirnauflageelement 2 benachbarten Auslaßöffnungen 6 ist bei der gezeigten Ausführungsform größer bemessen, als der Durchgangsquerschnitt der im Nasenspitzenbereich vorgesehenen Auslaßöffnungen 5. Das Größenverhältnis ist derart festgelegt, daß ca. 2/3 der ausströmenden Luft durch die dem Stimbereich benachbarten Auslaßöffnungen 6 und das verbleibende Drittel durch die vorderen Auslaßöffnungen 5 abströmt. Hierdurch wird ein optimaler Austausch der verbrauchten Atemluft bei geringen Zuglufterscheinungen erreicht.

Das Stirnauflageelement 2 ist derart ausgebildet, daß dieses großflächig und unter gleichmäßiger Flächenpressung auf dem Stirnbereich des Patienten aufliegt. Das Stirnauflageelement 2 ist mit Halteorganen 7 versehen, an welchen ein entsprechender Koppelungsabschnitt einer Maskenhalterung, insbesondere einer Kopfbandanordnung oder Halte-Mütze anbringbar ist. Eine bevorzugte Ausführungsform dieser Halteorgane 7 ist in Fig. 1c dargestellt. Der Maskenbasiskörper 1 ist derart ausgebildet, daß der Übergangsbereich zwischen dem Maskenbasiskörper und dem Dichtlippenelement einen im wesentlichen der Gesichtsform des Patienten folgenden Höhenverlauf aufweist. Insbesondere ist der Maskenbasiskörper im Bereich des Nasenrückens des Patienten derart eingezogen ausgebildet, daß das Dichtlippenelement - wie in Fig. 1e angedeutet - sattelartig über den Nasenrücken des Patienten geführt werden kann.

Das Dichtlippenelement 4 weist gem. einer bevorzugten Ausführungsform der Erfindung einen Querschnitt auf, der im wesentlichen der Darstellung gem. Fig. 1b entspricht. Das hier dargestellte Dichtlippenelement 4 umfaßt einen Schlauchpolsterkörper 8 und eine hier integral mit dem Schlauchkörper 8 ausgebildete Gesichtsdichtlippe 9. Die Gesichtsdichtlippe 9 weist hier im Bereich der Koppelungsstelle mit dem Schlauchpolsterkörper 8 eine vergleichsweise große Dicke t auf und verjüngt sich zunehmend zu einem Spitzenbereich s hin. In einem zwischen dem Polsterkörper 8 und dem Wurzelbereich der Gesichtsdichtlippe 9 gebildeten Übergangsbereich ist hier eine Rundung 10 ausgebildet, wodurch sich dieser zwischen der Gesichtsdichtlippe 9 und dem Schlauchpolsterkörper 8 gebildete Zwischenbereich verbessert reinigen läßt.

Der Schlauchpolsterkörper 8 ist im Bereich der außenseitigen Schlauchwandung 8a dickwandiger ausgebildet, als im Bereich der maskeninnenseitigen Schlauchwandung 8b. Der Übergang zwischen der Gesichtslippe 9 und der außenseitigen Schlauchpolsterwandung 8a ist derart gewellt, daß der räumliche Verlauf der Gesichtslippe 9 sowie des Schlauchpolsterkörpers 8 deutlich durch das Versteifungselement 3 beeinflußt werden kann. Das Versteifungselement 3 weist hier einen im wesentlichen rechteckförmigen Querschnitt auf und ist in einer Ausnehmung 11 aufgenommen, die unmittelbar dem Schlauchpolsterkörper 8 benachbart angeordnet ist. Bei der hier dargestellten Ausführungsform sind der Schlauchpolsterkörper 8 und der sich daran anschließende Maskenbasiskörper 1 integral aus ein und demselben Elastomermaterial ausgebildet. Die Ausnehmung 11 ist über einen Schlitzbereich 12 zur Außenseite der Atemmaske offen, so daß das Versteifungselement 3 bedarfsweise von dem Maskenbasiskörper 1 entfernt werden kann. Die Gesichtsdichtlippe 9 und der Schlauchpolsterkörper 8 sind derart angeordnet, daß die Andruckkraft der Gesichtsdichtlippe 9 durch den Schlauchpolsterkörper 8 noch weiter verstärkt wird, wenn der Maskenbasiskörper 1 mit einer vorbestimmten Anpreßkraft gegen das Gesicht des Patienten gedrängt wird.

Diese vorbestimmte Anpreßkraft wird vorzugsweise über eine Kopfbandanordnung erzeugt, die wenigstens ein um den Hinterkopfbereich eines Patienten herumgeführtes Gurtband aufweist. Dieses Gurtband kann über entsprechende Endstücke, bzw. Koppelungsorgane auf einfache Weise mit der Atemmaske über die bereits genannten Halteorgane 7 gekoppelt werden. Diese Halteorgane 7 können - wie in Fig. 1c dargestellt - integral mit dem Maskenbasiskörper 1, bzw. mit dessen Stirnauflageelement 2, ausgebildet sein. Im vorliegenden Falle weisen die Halteorgane einen Schaftabschnitt 13 und einen daran angeformten Kugelkopfabschnitt 14 auf. Der Durchmesser des Kopfabschnittes 14 ist größer als der Durchmesser des Schaftabschnittes 13. Derartige Halteorgane sind vorzugsweise an mehreren Stellen der Atemmaske 1, insbesondere auch im vorderen Bereich des Maskenbasiskörpers 1 - wie angedeutet - angeordnet.

Wie aus Fig. 1d hervorgeht, ist bei der dargestellten Atemmaske das Stirnauflageelement 2 derart langgestreckt ausgebildet, daß sich eine gleichmäßige Verteilung der Auflagekräfte ergibt. Das Stirnauflageelement 2 ist über einen Halsabschnitt mit dem Maskenbasiskörper 1 gekoppelt. Der Maskenbasiskörper 1, der Halsabschnitt 15 und das Stirnauflageelement 2 sind hier einstückig ausgebildet. Durch den Halsabschnitt 15 hindurch erstreckt sich ein Atemgaskanal 16, der bis in einen - ebenfalls mit dem Stirnauflageelement 2 ausgebildeten Anschlußabschnitt 17 mündet. Der Innendurchmesser des Anschlußabschnittes 17 ist derart ausgebildet, daß eine entsprechende Anschlußstruktur eines Atemgasschlauches unmittelbar in den Anschlußabschnitt 17 eingesteckt werden kann. Im Bereich des Anschlußabschnittes 17 sind auch die vorangehend bereits unter Bezugnahme auf Fig. 1 a erläuterten Auslaßöffnungen 6 vorgesehen, über welche ein vorbestimmter Gasstrom nach außen entweichen kann. Das Stirnauflageelement 2 weist bei der dargestellten Ausführungsform zwei Flügelabschnitte 18 auf, durch welche das Stirnauflageelement 2 noch weiter versteift wird, und zudem die über die Halteorgane 7 eingeleiteten Zugkräfte gleichmäßig übertragen werden. Auch die Flügelabschnitte 18 sind bei der dargestellten Ausführungsform einstückig mit dem Maskenbasiskörper, dem Halsabschnitt 15 und dem Stirnauflageelement 2 aus einem Elastomermaterial gebildet. Durch entsprechende Querschnittsgestaltung der Flügelabschnitte kann das Verformungsverhalten des Stimauflageeiementes gezielt beeinflußt werden.

In Fig. 1e ist lediglich andeutungsweise dargestellt, wie der Schlauchpolsterkörper 8 sattelartig dem Gesichtsprofil des Patienten folgend über den Nasenrücken herumgeführt ist. Der Anpreßdruck des Schlauchpolsterkörpers 8 im Bereich des Nasenrückens kann auf vorteilhafte Weise durch das Versteifungselement 3 beeinflußt werden, indem die hier andeutungsweise skizzierte Nasenrückenweite n durch das Versteifungselement 3 definiert festgelegt wird. Hierdurch wird vermieden, daß der Nasenrückenbereich des Patienten über den Schlauchpolsterkörper 8 in unangenehmer Weise zu stark zusammengedrückt wird. Durch die Kombination von Schlauchkörper 8 und Versteifungselement 3 wird es auf vorteilhafte Weise möglich, einen besonders hohen Tragekomfort sowie eine hohe Dichtigkeit der Maske zu erreichen, da der Schlauchpolsterkörper 8 vor Auflage auf der Gesichtsfläche des Patienten bereits in eine weitgehend der Gesichtskontur des Patienten entsprechende Gestalt aufweist. Die Kombination von Versteifungskörper 3 und Schlauchpolsterkörper 8 kann auch unabhängig von der integralen Ausbildung von Maskenbasiskörper 1 und Stirnauflageelement 2 Anwendung finden.

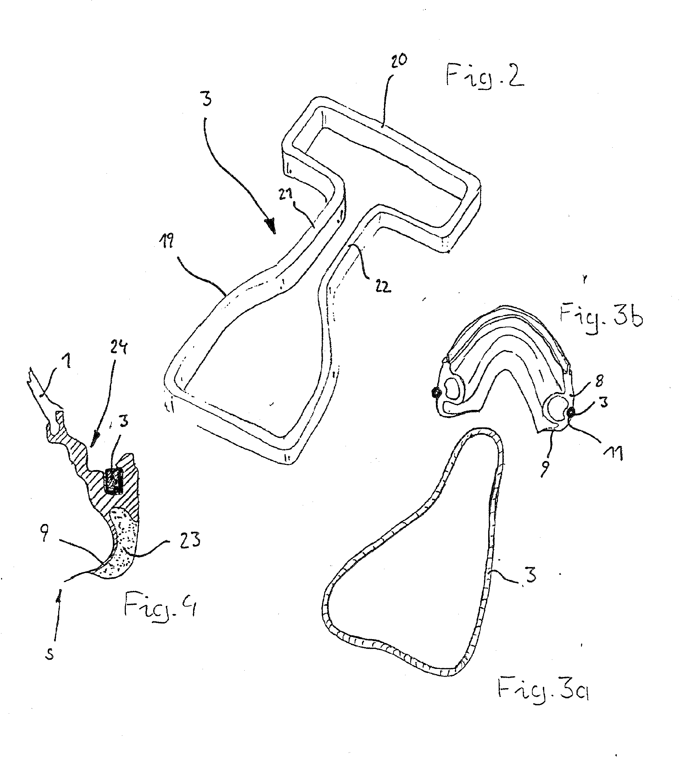

In Fig. 2 ist eine bevorzugte Ausführungsform des Versteifungselementes 3 dargestellt, das hier sowohl einen der Gesichtskontur des Patienten im Bereich der Nase angepaßten, bzw. anpaßbaren Abschnitt 19 sowie einen an die Stirnkontur des Patienten angepaßten Abschnitt 20 aufweist.

Die beiden Abschnitte 19, 20 sind über Schenkel 21, 22 miteinander gekoppelt. Das Versteifungselement 3 ist hier aus einem thermoverformbaren Kunststoffmaterial gebildet, das gemeinsam mit einem entsprechenden Maskenbasiskörper mit integralem Stirnauflageelement an den Patienten angepaßt wird. Nach Erkalten des entsprechenden Kunststoffmaterials behält das Versteifungselement 3 die ihm individuell gegebene Form dauerhaft bei. Das Versteifungselement 3 kann bedarfsweise von dem Maskenbasiskörper 1 und dem Stirnauflageelement 2 (vgl. hierzu Fig. 1a) entfernt werden und in einen neuen entsprechenden Maskenbasiskörper eingesetzt werden, so daß dieser im wesentlichen die gleiche Gestalt wie der ursprüngliche Maskenkörper erhält. Durch die hier dargestellte Ausführungsform des Versteifungselementes 3 wird es möglich, insbesondere das Höhenniveau des Stirnauflageelementes und des Maskenbasiskörpers 1 exakt aufeinander abzustimmen, so daß die entsprechend individuell vorgeformte Atemmaske bereits vor Aufsetzen auf das Gesicht des Patienten eine weitgehend dessen Gesichtskontur entsprechende Gestalt aufweist.

Alternativ zu der in Fig. 2 beschriebenen Ausführungsform des rahmenartig ausgebildeten Versteifungselementes 3 ist es auch möglich, dieses beispielsweise aus einem Drahtmaterial zu fertigen, wie in Fig. 3a vereinfacht angedeutet. Hierbei wird vorzugsweise ein nicht korrodierender Stahlwerkstoff verwendet, der über entsprechende Biegewerkzeuge in die gewünschte Form gebogen wird. Dieses durch ein Drahtbügel gebildete Versteifungselement 3 kann dann, wie in Fig. 3b angedeutet, mit dem Maskenbasiskörper 1 bzw. mit dessen Dichtungsstruktur gekoppelt werden. Vorzugsweise weist der Maskenbasiskörper 1, bzw. die Dichtungsstruktur 8, 9 entsprechende Ausnehmungen 11 auf, in die das Versteifungselement 3 eingefügt werden kann. Bei der hier in Fig. 3b andeutungsweise darge-stellten Dichtungsstruktur ist lediglich der über den Nasenrücken des Patienten geführte Dichtungsstrukturbereich dargestellt.

In Fig. 4 ist eine weitere bevorzugte Ausführungsform einer Dichtungsstruktur für eine Atemmaske dargestellt, die hier eine Gesichtsdichtlippe 9 aufweist, die zu einer äußerst dünnen Dichtlippenspitze s ausläuft. Die Wandungsdicke der Gesichtsdichtlippe 9 nimmt zu einem Wurzelbereich der Gesichtsdichtlippe hin allmählich zu. An den Außenbereich der Gesichtsdichtlippe 9 ist ein Polsterkörper 23 angeformt, der hier aus einem gelartig ausgehärteten Material - hier Silikonkautschuk mit niedrigem Vernetzungsgrad - gebildet ist. Dieser Polsterkörper 23 stützt den Maskenbasiskörper 1 gemeinsam mit der Dichtlippe 9 auf dem Gesicht des Patienten ab. Bei der hier dargestellten Ausführungsform ist der Maskenbasiskörper 1 separat von der Dichtungsstruktur 24 ausgebildet. Die Dichtungsstruktur ist ähnlich wie bei den vorangehend beschriebenen Ausführungsformen über ein Versteifungselement 3 bereits in eine der jeweiligen Gesichtskontur des Patienten entsprechende räumliche Gestalt vorgeformt. Es ist auch möglich, die hier gezeigte Dichtungsstruktur 24 integral mit dem Maskenbasiskörper 1 auszubilden. Der Außenbereich des Polsterkörpers 23 ist mit einer Hautschicht überzogen, die ein Ankleben des Polsterkörpers 23 an der Gesichtsoberfläche des Patienten verhindert. Die Oberfläche dieser Hautschicht weist hier eine vorbestimmte samtartige Mikrorauhigkeit auf.

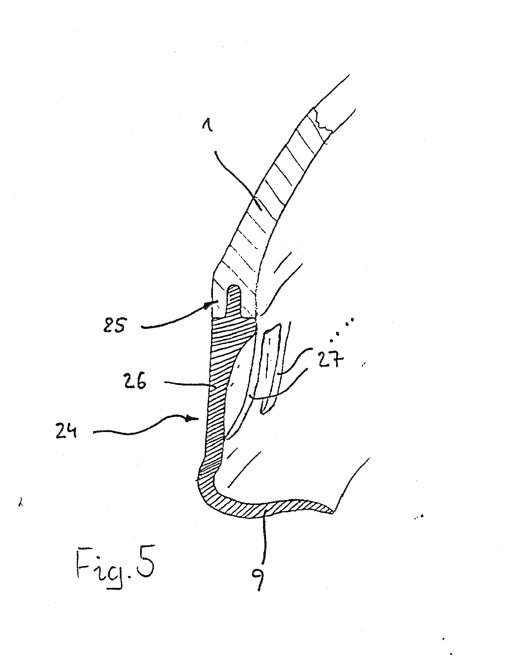

In Fig. 5 ist eine weitere Ausführungsform einer Dichtungsstruktur 24 für eine Atemmaske dargestellt, die hier ebenfalls eine vom Außenbereich der Atemmaske sich im wesentlichen radial einwärts erstreckende dünn auslaufende Gesichtsdichtlippe 9 aufweist. Der Krümmungsverlauf der Gesichtsdichtlippe 9 in der gezeigten Schnittebene (Ebene π in Fig. 1a) ist derart gewählt, daß sich die Dichtlippe vergleichsweise gut an das Profil des Patienten anpassen kann. Die Dichtungsstruktur 24 und der Maskenbasiskörper 1 sind hier aus unterschiedlichen Werkstoffen gebildet. So ist bei dem gezeigten Ausführungsbeispiel die Dichtungsstruktur aus einem Silikonmaterial und der Maskenbasiskörper aus einem thermoplastischen Kunststoffmaterial gebildet. Der Maskenbasiskörper 1 ist in einem speziellen Formwerkzeug unmittelbar an die im Rahmen eines vorangegangenen Silikonspritzschrittes gebildete Dichtlippenstruktur angespritzt, wodurch eine im wesentlichen unlösbare und spaltfreie Verbindung zwischen der Dichtungsstruktur 24 und dem Maskenbasiskörper erreicht wird. Bei der gezeigten Ausführungsform ist im Bereich der Übergangsstelle zwischen der Dichtungsstruktur 24 und dem Maskenbasiskörper ein umlaufender Stegbereich 25 vorgesehen, über weichen die Festigkeit der Verbindung zwischen Maskenbasiskörper und Dichtungsstruktur 24 noch weiter verbessert wird.

Das Verformungsverhalten der Dichtungsstruktur 24 wird durch eine definierte Gestaltung der Wanddicken der Gesichtsdichtlippe 9 sowie des sich daran anschließenden, zum Maskenbasiskörper 1 fortsetzenden Wandungsabschnitt 26 definiert beeinflußt. Weiterhin sind zahlreiche integral mit der Dichtungsstruktur 24 ausgebildete Stege 27 vorgesehen, deren Länge und Wandstärke ebenfalls das Verformungsverhalten der Dichtungsstruktur 24 definiert beeinflußt. Obgleich bei der hier dargestellten Ausführungsform kein Versteifungselement vorgesehen ist, ist es auch möglich, bei dieser Dichtungsvariante mittels des Versteifungselementes eine noch individuellere Anpassung an die Gesichtskontur des Patienten zu erreichen.

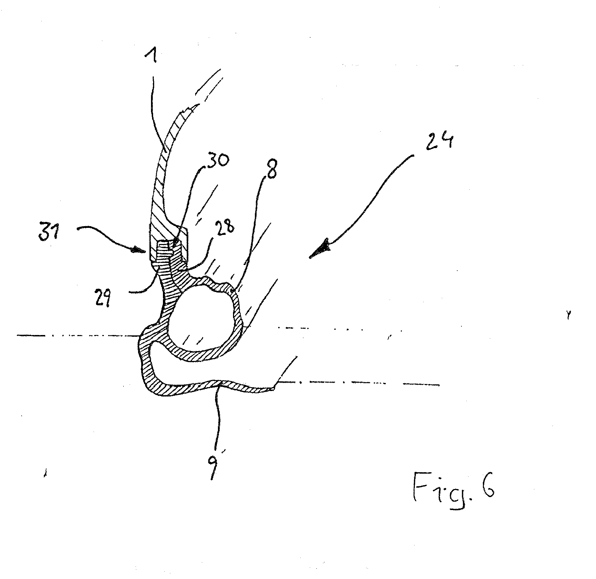

Fig. 6 ist eine weitere bevorzugte Ausführungsform einer Dichtungsstruktur 24 dargestellt, die hier ebenfalls einen Schlauchpolsterkörper 8 und eine sich radial einwärts erstreckende Gesichtsdichtlippe 9 aufweist. Die Wandung sowohl der Gesichtsdichtlippe 9 als auch des Schlauchpolsterkörpers 8 ist hier derart definiert dick- bzw. dünnwandig ausgebildet, daß die Dichtungsstruktur eine im Hinblick auf eine besonders gleichmäßige Gesichtsflächenpressung vorteilhafte Mechanik erhält. Durch die dünnwandigen Abschnitte der Dichtlippe 9 und des Schlauchpolsterkörpers 8 kann sich die Dichtungsstruktur gleichmäßig an das Gesicht des Patienten anschmiegen. Der Schlauchpolsterkörper 8 ist unter Anwendung eines Formkernes gebildet, der über eine ursprünglich zwischen zwei Flankenabschnitten 28, 29 gebildete Öffnung entformt wurde. Die beiden Flankenabschnitte 28, 29 sind miteinander verklebt. Um ein definierte Positionierung der beiden Flankenabschnitte 28, 29 zu einander zu erhalten, sind hier entsprechende Profilierungen, beispielsweise eine Nut/Feder-Profilierung 30 vorgesehen. Die zwischen den beiden Flankenabschnitten 28, 29 gebildete Fügestelle 30 wird von einem Aufnahmerand 31 des Maskenbasiskörpers 1 übergriffen und ist damit von außen nicht erkennbar und zudem, insbesondere während der Aushärtung eines Klebstoffmaterials wirkungsvoll geschützt.

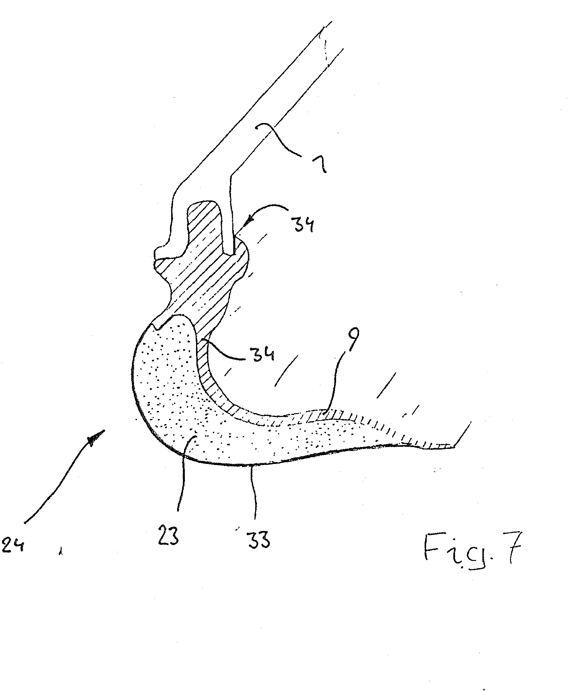

In Fig. 7 ist eine weitere bevorzugte Ausführungsform einer Dichtungsstruktur für eine Atemmaske gezeigt, die ähnlich, wie die bereits in Verbindung mit Fig. 4 beschriebene Dichtungsstruktur eine zur Gesichtsfläche des Patienten hin dünn auslaufende Gesichtsdichtlippe 9 aufweist. Diese Gesichtsdichtlippe 9 weist eine vergleichsweise hohe Tragfähigkeit auf. Die der Gesichtsfläche des Patienten zugewandte Außenseite der Gesichtsdichtlippe 9 ist zum überwiegenden Teil mit einem Polsterkörper 23 überzogen, der aus einem Elastomermaterial mit niedrigem Vernetzungsgrad gebildet ist (Gelmaterial). Die Außenseite dieses Polsterkörpers 23 ist mit einer samtartig rauhen dünnen Hautschicht 33 überzogen. Diese dünne Hautschicht 33 ist gem. einer besonders bevorzugten Ausführungsform der Erfindung aus einem Elastomermaterial, insbesondere ebenfalls Silikonmaterial mit höherem Vernetzungsgrad gebildet. Hierdurch wird es möglich, den Gelkörper zu verformen, ohne, daß an dessen Außenseite Kräuselfalten auftreten. Der Querschnitt der Dichtungsstruktur 24 ist derart gewählt, daß ein Teil der über die dünne Hautschicht 33 in dem Polsterkörper 23 eingeleiteten Aufstandskräfte sich unmittelbar in einen Wurzelbereich 24 der Gesichtsdichtlippe 9 von hier aus in den Maskenbasiskörper 1 überträgt. Der Maskenbasiskörper ist bei der hier dargestellten Ausführungsform mit der Dichtungsstruktur 24 über eine Nut/Federstruktur verklebt. Eine innere Fügungsstelle 34 zwischen dem Maskenbasiskörper 1 und der Dichtungsstruktur 24 ist durch eine an die Dichtungsstruktur 24 angeformte Nase abgedichtet.

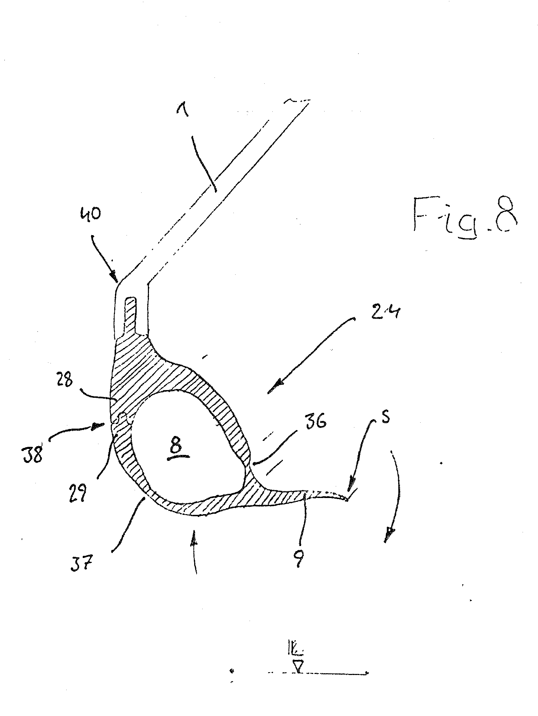

In Fig. 8 ist eine weitere bevorzugte Ausführungsform einer Dichtungsstruktur 24 dargestellt, die hier ebenfalls einen Schlauchpolsterkörper 8 mit integral angeformter Gesichtsdichtlippe 9 aufweist. An dem Schlauchpolsterkörper 8 sind wenigstens zwei sich über den gesamten Umfang der Dichtungsstruktur erstreckende dünnwandige Abschnitte 36, 37 vorgesehen, durch welche eine Kippzone gebildet wird, über welche die Gesichtsdichtlippe 9 tendenziell zunächst mit ihrem Spitzenbereich f an die Gesichtsfläche des Patienten gedrängt wird. Der übrige Bereich des Schlauchpolsterkörpers 8 ist vergleichsweise dickwandig ausgebildet. Der Schlauchpolsterkörper 8 wird auch bei der hier dargestellten Ausführungsform unter Verwendung eines Formkernelements hergestellt, das über eine hier andeutungsweise dargestellte Fügestelle 38 entformt wird. Im Bereich der Fügestelle 38 sind die entsprechend benachbarten Abschnitte 28, 29 miteinander verklebt. Eine definierte Positionierung der beiden Abschnitte 28, 29 wird auch hier, wie bereits in Verbindung mit Fig. 6 beschrieben, durch eine entsprechende Profilierung erreicht.

Der Schlauchpolsterkörper 8, bzw. die gesamte Dichtungsstruktur 24 ist hier aus einem extrem weichen Silikonkautschukmaterial gebildet, das sich unter vergleichsweise geringen Verformungskräften an die Gesichtskontur des Patienten hochelastisch anpaßt. Die hohe Dichtwirkung dieser Dichtungsstruktur wird dadurch erreicht, daß über den hier verwirklichten Gelenkmechanismus über die Gesichtsauflagekräfte erreicht wird, daß der Spitzenbereich s der Gesichtsdichtlippe 9 sich durchgängig an die Gesichsoberfläche des Patienten anschmiegt. Der Maskenbasiskörper 1 ist bei der dargestellten Ausführungsform über eine entsprechende Falzverbindungsstelle 40 mit der Dichtungsstruktur 24 verklebt. Ggf. kann auch eine rollbalgartige Nachgiebigkeit der Dichtungsstruktur realisiert werden.

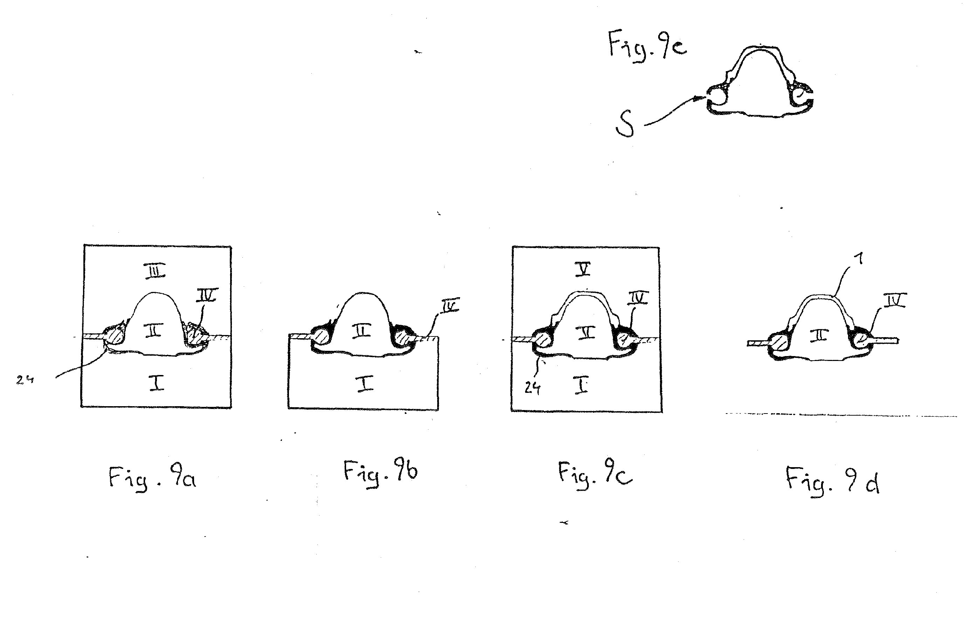

Unter Bezugnahme auf die Fig. 9a bis 9e wird nachfolgend der Herstellungsvorgang einer Atemmaske beschrieben, die eine aus einem Elastomermaterial gebildete Dichtungsstruktur mit einem Luftpolster sowie einen aus einem thermoplastischen Kunststoffmaterial gebildeten Maskenbasiskörper aufweist.

Wie in Fig. 9a dargestellt, wird hierzu zunächst eine Formwerkzeuganordnung geschaffen, die zur Durchführung eines ersten Herstellungsschrittes ausschließlich den zur Bildung der Dichtungsstruktur 24 erforderlichen Formraum begrenzt. Diese Werkzeuganordnung umfaßt hierbei ein Untergesenk I, einen Maskenkern II, ein Formobergesenk III und einen Luftpolsterkern IV.

In den derart begrenzten Formraum wird ein Silikonmaterial eingespritzt. Nachdem dieses Silikonmaterial unter Bildung der Dichtungsstruktur 24 ausgehärtet ist, wird das Formoberwerkzeug 3 abgenommen.

Die Dichtungsstruktur verbleibt weiterhin in dem unteren Formwerkzeug. Auch die beiden Formkeme II und IV werden nicht aus der Dichtungsstruktur 24 entfernt, wie dies in Fig. 9b dargestellt ist.

Wie in Fig. 9c dargestellt, wird nunmehr ein weiteres Formoberwerkzeug V auf die Anordnung nach Fig. 9b aufgesetzt. Das Formoberwerkzeug V begrenzt gemeinsam mit dem Formkem II und einem Teil der vorab gebildeten Dichtungsstruktur einen zur Bildung des Maskenbasiskörpers vorgesehenen Formraum. In diesen Formraum wird nunmehr ebenfalls ein Kunststoffmaterial, insbesondere ein thermoplastisches Kunststoffmaterial eingespritzt, das mit der Dichtungsstruktur 24 eine innige Verbindung eingeht.

Nach Erhärten dieses Kunststoffmateriales wird das Formoberwerkzeug V abgenommen. Nunmehr kann die Dichtungsstruktur 24 mit dem angespritzten Maskenbasiskörper 1 aus dem Untergesenk I entnommen werden.

Die Entformung des Luftpolsterkemes und des Formkernes II erfolgt manuell ggf. unter Zuhilfenahme von Druckluft. Die hohe Eigenelastizität der Dichtungsstruktur 24 erlaubt auch bei dem hier vorhandenen großen Hinterschnitt eine einfache Entformung.

Bei der hier gezeigten Ausführungsform wird zunächst der Formkem II und erst dann der Luftpolsterformkern IV entformt. Hierbei erhält man die in Fig. 9e dargestellte Maske. Der hier noch offene, umlaufende Entformungsspalt S kann nunmehr unter Zuhilfenahme eines Klebemittels geschlossen werden.

## Patentansprüche

1. Atemmaske mit:
- einem Maskenbasiskörper der einen Maskeninnenraum begrenzt,
- einem Atemgaszuleitungsabschnitt zur Zuleitung von Atemgas zu dem Maskeninnenraum, und
- einer nachgiebigen Dichtungseinrichtung zur Abdichtung des Maskenbasiskörpers, wobei die Dichtungseinrichtung einen aus einem gelartig ausgehärteten Silikonmaterial gebildeten Abschnitt aufweist, **dadurch gekennzeichnet, dass** der Silikonmaterial-Abschnitt mit einer Hautschicht überzogen ist die aus einem Silikonmaterial gebildet ist.

2. Atemmaske nach Anspruch 1, **dadurch gekennzeichnet, dass** die Hautschicht aus einem höher vernetzten Silikonkautschukmaterial gebildet ist.

3. Atemmaske nach einem der vorangegangenen Ansprüche, wobei der Gelabschnitt bzw. Gelkörper verformbar ist, ohne dass an dessen Außenseite Kräuselfalten auftreten.

4. Atemmaske nach einem der vorangegangenen Ansprüche, **dadurch gekennzeichnet, dass** die Hautschicht durch Auftrag einer dünnen Silikonmaterialschicht auf einen entsprechenden Formraumabschnitt eines Formwerkzeuges gebildet ist, und dass das gelartig aushärtende Silikonmaterial in den entsprechend vorbereiteten Formraumabschnitt eingespritzt ist, und/oder wobei das Polsterorgan zwei Flankenabschnitte aufweist, die miteinander verklebt sind.

5. Atemmaske nach wenigstens einem der vorangehenden Ansprüche, wobei die Dichtungseinrichtung ein Dichtlippenelement aufweist das aus einem Silikonmaterial mit hohem Vemetzungsgrad gefertigt ist, und dass an dieses Dichtlippenelement der Gelkörper angekoppelt ist, der aus einem Silikonmaterial niedrigen Vernetzungsgrades gefertigt ist, und/oder wobei eine einstückig mit dem Polsterorgan ausgebildete Dichtlippe vorgesehen ist die sich vom Maskenaussenbereich einwärts erstreckt.

6. Atemmaske nach wenigstens einem der vorangehenden Ansprüche, wobei der Gelkörper ein aus einem elastomeren Material gebildetes Schlauch-Polsterelement ist und wobei, vorzugsweise, Wandungsabschnitte des Schlauchpolsters derart definiert dick- und dünnwandig ausgebildet sind, dass definierte Gelenkzonen entstehen.

7. Atemmaske nach wenigstens einem der vorangehenden Ansprüche, wobei ein einwärts gerichtet umlaufendes Dichtlippenelement einstückig mit dem Polsterkörper ausgebildet ist.

8. Atemmaske nach wenigstens einem der vorangehenden Ansprüche, wobei die Dichtungseinrichtung aus einem Silikonmaterial gebildet ist.

9. Atemmaske nach wenigstens einem der vorangehenden Ansprüche, wobei der Maskenbasiskörper an die Dichtungseinrichtung angespritzt ist.

10. Atemmaske nach wenigstens einem der vorangehenden Ansprüche, wobei die Maske ein Stirnauflagelement aufweist, wobei das Stirnauflageelement vorzugsweise mit einer Koppelungseinrichtung versehen ist, zum Anschluss eines Maskenhaltebandes.

11. Atemmaske nach wenigstens einem der vorangehenden Ansprüche, wobei das Polsterorgan sich in der Art eines Schlauchpolsters entlang des Maskenauflagebereiches erstreckt.

12. Atemmaske nach wenigstens einem der vorangehenden Ansprüche, wobei ein Innenübergangsbereich zwischen der Dichtlippe und dem Polsterorgan gerundet ausgebildet ist.

13. Atemmaske nach wenigstens einem der vorangehenden Ansprüche, wobei der Maskenbasiskörper separat von der Dichtungseinrichtung ausgebildet ist.

14. Atemmaske nach wenigstens einem der vorangehenden Ansprüche, wobei der Maskenbasiskörper mit der Dichtungseinrichtung über eine Nut/Federstruktur verbunden ist.
